# EUROPEAN PATENT APPLICATION

(11) **EP 4 628 511 A1**
(43) Date of publication of application: **08.10.2025**
(21) Application number: 23896880.4
(22) Date of filing: 30.11.2023
(51) Int. Cl.: C07K 19/00, C12N 15/11

(54) **RECOMBINANT HEMOGLOBIN**

(30) Priority: 30.11.2022 CN 202211528623
(71) Applicant: Kangma-Healthcode (Shanghai) Biotech Co., Ltd, Pudong New Area Shanghai 201321 (CN)
(72) Inventor: GUO, Min, Shanghai 201321 (CN); XU, Liqiong, Shanghai 201321 (CN); WANG, Songlin, Shanghai 201321 (CN); LIU, Zhang, Shanghai 201321 (CN); XIE, Gaowei, Shanghai 201321 (CN); XIAO, Song, Shanghai 201321 (CN); YU, Xue, Shanghai 201321 (CN)
(74) Representative: Murgitroyd & Company
(86) International application number: PCT/CN2023/135487
(87) International publication number: WO 2024/114738

(57) **Abstract**

Provided in the present invention are a recombinant hemoglobin, a nucleic acid encoding the recombinant hemoglobin, a preparation method therefor, etc., so as to solve the current problems of complex processes, high production costs, low yields, etc. that are present in the process of improving the stability of the tetrameric structure of hemoglobin. The recombinant hemoglobin comprises four polypeptide chains, the polypeptide chains being two α chains and two β chains respectively, wherein the amino acid sequences of the four polypeptide chains are linked by means of a linker peptide, and the linker peptides are preferably used to sequentially link the amino acid sequences of the four polypeptide chains from the N terminus to the C terminus in any order.

## Description

### TECHNICAL FIELD

The present disclosure belongs to the field of biotechnology, and specifically relates to a recombinant hemoglobin, a nucleic acid encoding the recombinant hemoglobin, a preparation method therefor, and the like.

### BACKGROUND

Hemoglobin is a protein with oxygen-carrying function in red blood cells. Hemoglobin has a spherical structure, and the molecular weight of natural hemoglobin is 64.5 KD. Hemoglobin is composed of four polypeptide chains (subunits) that are bound together by non-covalent interactions. The main component of human whole blood is hemoglobin A, with a structure of α₂β₂, in which the α chain contains 141 amino acid residues and the β chain contains 146 amino acid residues. The α chain and the β chain are tightly bound to form a dimer, and two αβ dimers are loosely bound to form a hemoglobin molecule.

Blood products (Blood products are defined as any therapeutic substance extracted from human blood, including whole blood, labile blood components and plasma-derived medicinal products.) can save millions of lives every year, greatly increase life expectancy and improve the quality of life of patients at risk of death, and support complex medical and surgical procedures.

However, blood supply services worldwide face huge challenges, requiring sufficient blood product supplies to meet patient needs while ensuring their quality and safety in the face of known and emerging public health threats. According to the statistics of 2002, more than 750 million units of blood were donated worldwide every year, and only 43 blood donors in the World Health Organization (191 member countries in total) have been tested for hepatitis B virus (HBV), hepatitis C virus (HCV) and human immunodeficiency virus (HIV). Unsafe blood transfusions or injections cause 80,000 to 160,000 people to be infected with HBV, 23,000 to 47,000 people to be infected with HCV, and 80,000 to 160,000 people to be infected with HIV every year. In addition, the contradiction between the increased blood demand and the decreased blood donation, hemolysis or even death caused by incorrect blood transfusion, the short storage time of human red blood cells, natural disasters, wars and other reasons have forced people to look for a red blood cell substitute that can replace blood.

Hemoglobin-based red blood cell substitutes are hemoglobin-based biological drugs with oxygen-carrying function. Hemoglobin-based red blood cell substitutes can be divided into three categories, namely, red blood cell substitutes based on human blood, red blood cell substitutes based on animal blood, and red blood cell substitutes based on recombinant hemoglobin.

However, currently, unmodified hemoglobin cannot be used as a blood substitute. One of the main reasons is that free hemoglobin tetramers can be degraded into αβ dimers and monomers, causing many side effects. In order to reduce side effects, stabilizing hemoglobin tetramers is a necessary condition for hemoglobin to be used as a blood substitute. At present, chemical modification methods, such as cross-linking, polymerization and coupling, are often used to increase the molecular weight of hemoglobin. In addition to chemical modification to obtain the product, biotechnology, such as site-directed mutation at the DNA level, can also achieve the effect of stabilizing hemoglobin tetramer.

However, although the chemical modification method is relatively mature, there are also side effects that need to be solved urgently. (1) Due to the different chemical reagents and methods used and the non-specificity of the cross-linking reaction, the molecular size, oxygen affinity, subunit synergy, stability and toxic side effects of hemoglobin products are difficult to control, and the final product is a non-uniform mixture. (2) The contamination of pathogenic microorganisms still exists because the problem of blood disinfection has not been solved, and there is still a risk of spreading blood-borne diseases such as mad cow disease and acute and chronic immunogenicity to humans. (4) The instability of the final product leads to the rearrangement of cross-linked hemoglobin. Because free cross-linking agents are cytotoxic, whether they will fall off from cross-linked hemoglobin is an issue worthy of attention. In addition, chemical modification sometimes also involves complex processes, which increases production costs and reduces production efficiency.

However, site-directed mutation of hemoglobin genes at the DNA level can sometimes lead to blindness, thus affecting the final function.

In addition, there are also methods for producing recombinant hemoglobin by co-expressing α and β in cells, but the yield is often very low, and after production, cross-linking chemical modification is required to increase the stability of the tetrameric structure.

It can be seen that there are deficiencies in the prior art for providing hemoglobin with a stabilized tetrameric structure.

### SUMMARY

The present disclosure provides a recombinant hemoglobin, a nucleic acid encoding the recombinant hemoglobin, a preparation method therefor and the like, in order to solve the problems of complex processes, high production cost, large side effects, low yield and the like in order to improve the stability of hemoglobin with a tetrameric structure.

Therefore, the present disclosure provides the following technical solutions:

The present disclosure provides a recombinant hemoglobin, characterized in that the recombinant hemoglobin comprises: four polypeptide chains, and the polypeptide chains are two α chains and two β chains, respectively, wherein the amino acid sequences of the four polypeptide chains are linked by a linker peptide.

The recombinant hemoglobin provided by the present disclosure is further characterized in that: wherein three of the linker peptides sequentially link the amino acid sequences of the four polypeptide chains from N-terminus to C-terminus in any order.

The recombinant hemoglobin provided by the present disclosure is further characterized in that: the number of amino acids in the linker peptide is 1-90, 1-30, 1-35 or 1-40.

The recombinant hemoglobin provided by the present disclosure is further characterized in that: the linker peptide used to link the two α chains is a first linker peptide, and the number of amino acids in the first linker peptide is 1-5, preferably 1, 2 or 3; the linker peptide used to link the two β chains or one α chain and one β chain is a second linker peptide, and the number of amino acids in the second linker peptide is 5-90, 5-85, 5-30, 5-35, 5-40, 10-30, 10-35 or 10-40.

The recombinant hemoglobin provided by the present disclosure is further characterized in that: the amino acid of the first linker peptide is 1 glycine.

The recombinant hemoglobin provided by the present disclosure is further characterized in that: the second linker peptide comprises main amino acid residues selected from one or more of G, S, T and A, and the number of the main amino acid residues accounts for at least greater than 50%, 60%, 70%, 80% or 90% of the total number of amino acid residues of the second linker peptide; more preferably, the second linker peptide comprises main amino acid residues selected from one or more of G and S, and the total amount of the main amino acid residues accounts for at least greater than 40%, 45%, 60%, 70% or 80% of the total number of amino acid residues of the second linker peptide.

The recombinant hemoglobin provided by the present disclosure is further characterized in that: the second linker peptide comprises at least a flexible unit.

The recombinant hemoglobin provided by the present disclosure is further characterized in that: the second linker peptide further comprises an amphipathic unit composed of alternating hydrophilic and hydrophobic amino acids; at this time, from N-terminus to C-terminus, the amino acid order of the second linker peptide is: flexible unit-amphipathic unit composed of alternating hydrophilic and hydrophobic amino acids-flexible unit.

The recombinant hemoglobin provided by the present disclosure is further characterized in that: the structure of the flexible unit is (GGGGS)a, preferably, a is 1-4,
more preferably, the flexible unit is selected from:
(1) GGGGS;
(2) GGGGSGGGGS;
(3) GGGGSGGGGSGGGGSGGGGS.

The recombinant hemoglobin provided by the present disclosure is further characterized in that: the structure of the amphipathic unit is any one or more of (RADA)b, (KLDL)c, (LK)d and (LD)f, preferably: b is 1-5, c is 1-5, d is 1-10, and f is 1-10,
more preferably, the amphipathic unit is selected from:
(1) RADARADARADARADA;
(2) KLDLKLDLKLDL;
(3) LKLKLKLKLKLK;
(4) LDLDLDLDLDLD.

The recombinant hemoglobin provided by the present disclosure is further characterized in that: the amino acid sequences of the second linker peptide are SEQ ID NOs: 1-6.

The recombinant hemoglobin provided by the present disclosure is further characterized in that: the amino acid sequences of the four polypeptide chains are linked from N-terminus to C-terminus in any one of the following orders: α chain-α chain-β chain-β chain, β chain-β chain-α chain-α chain, β chain-α chain-α chain-β chain, α chain-β chain-α chain-β chain and β chain-α chain-β chain-α chain.

The recombinant hemoglobin provided by the present disclosure is further characterized in that: the α chain is derived from any one or more of human, pig or bovine species, and the β chain is derived from any one or more of human, pig or bovine species; and/or
the amino acid sequence of the α chain is any one or more of SEQ ID NOs: 7, 13 and 14, or has a percent sequence homology of at least 80%, 85%, 90%, 95%, 97% or 99% compared with any one or more of SEQ ID NOs: 7, 13 and 14, respectively;
the amino acid sequence of the β chain is any one or more of SEQ ID NOs: 8, 15 and 16, or has a percent sequence homology of at least 80%, 85%, 90%, 95%, 97% or 99% compared with any one or more of SEQ ID NOs: 8, 15 and 16, respectively.

The recombinant hemoglobin provided by the present disclosure is further characterized in that: the recombinant hemoglobin further comprises hemin bound to each polypeptide chain respectively.

The present disclosure further provides a nucleic acid, characterized in that the nucleic acid comprises: a nucleotide encoding the above recombinant hemoglobin.

The present disclosure further provides a vector, characterized in that the vector comprises: the above nucleic acid.

The present disclosure further provides a host cell, characterized in that the host cell comprises: the above nucleic acid and/or the above vector,
preferably, the host cell is derived from a prokaryotic cell or a eukaryotic cell, further, the host cell is derived from bacteria, mammalian cells, human cells, plant cells, yeast cells or insect cells, preferably selected from *Escherichia coli* or yeast cells, and furthermore, the yeast cell is selected from: one or more of *Saccharomyces cerevisiae* and *Kluyveromyces;* in another preferred embodiment, the *Kluyveromyces* is selected from: one or more of *Kluyveromyces lactis, Kluyveromyces marxianus* and *Kluyveromyces dobzhanskii.*

A use of the above nucleic acid, vector or host cell of the present disclosure in the preparation of the above recombinant hemoglobin.

The present disclosure further provides an *in vitro* cell-free protein synthesis system, characterized in that the system comprises: a cell extract; and an mRNA or DNA template encoding the above recombinant hemoglobin, preferably, the cell extract is an *Escherichia coli* extract or a yeast cell extract, further, the cell extract is derived from the following group: one or more of *Saccharomyces cerevisiae, Pichia pastoris* and *Kluyveromyces;* preferably, the yeast cell extract is derived from *Kluyveromyces,* more preferably one or more of *Kluyveromyces lactis, Kluyveromyces marxianus* and *Kluyveromyces dobzhanskii;*
furthermore, further comprising one or more of the following components:
an amino acid mixture, a dNTP, an RNA polymerase, a DNA polymerase, an energy supply system, polyethylene glycol and an aqueous solvent.

The present disclosure further provides an *in vitro* cell-free synthesis method for the above recombinant hemoglobin, characterized in that the method comprises: providing an *in vitro* cell-free protein synthesis system;
adding an mRNA or DNA template encoding the above recombinant hemoglobin to the *in vitro* cell-free protein synthesis system to perform an *in vitro* synthesis reaction to obtain the recombinant hemoglobin, preferably, the volume ratio of the DNA template to the *in vitro* cell-free protein synthesis system is 1:10 to 1:50, preferably 1:20 to 1:40, most preferably 1:25 to 1:35, particularly preferably 1:30.

The *in vitro* cell-free synthesis method provided by the present disclosure is further characterized in that the method further comprises: adding hemin to the *in vitro* cell-free protein synthesis system to generate recombinant hemoglobin bound to the hemin, and the order of adding the hemin is not limited, and may be added before or after the *in vitro* synthesis using the *in vitro* cell-free protein synthesis system.

Preferably, the added hemin comprises trivalent iron.

The *in vitro* cell-free synthesis method provided by the present disclosure is further characterized in that: wherein the *in vitro* cell-free protein synthesis system comprises: a cell extract;
further,
the *in vitro* cell protein synthesis system comprises one or more of an amino acid mixture, a dNTP, an RNA polymerase, a DNA polymerase, an energy supply system, polyethylene glycol and an aqueous solvent; and/or
the cell extract is an *Escherichia coli* extract or a yeast cell extract, further, the cell extract is derived from the following group: one or more of *Saccharomyces cerevisiae, Pichia pastoris* and *Kluyveromyces;* preferably, the yeast cell extract is derived from *Kluyveromyces,* more preferably one or more of *Kluyveromyces lactis, Kluyveromyces marxianus* and *Kluyveromyces dobzhanskii;* or
the yeast cell extract accounts for 50-80% of the entire *in vitro* cell-free protein synthesis system.

The present disclosure further provides the above recombinant hemoglobin for use as a blood substitute, and for use in cancer, stroke, hemorrhagic shock, acute mountain sickness (AMS), peripheral arterial disease (PAD) and Parkinson's disease (PD).

### Function and effect of the present disclosure

The recombinant hemoglobin, the nucleic acid encoding the recombinant hemoglobin and the preparation method therefor provided by the present disclosure have at least the following advantages:
(1) Recombinant hemoglobin links four polypeptide chains in tandem in the primary structure through a linker peptide, so that in the process of synthesizing and expressing recombinant hemoglobin *in vivo* or *in vitro,* the tandem α chains and β chains are more likely to interact with each other to form a monomer structure with similar functions to tetrameric hemoglobin, and due to the linkage in the primary structure, this structure will be more stable and not easy to dissociate, and can form a stable tetramer. In addition, the tandem α chains and β chains maintain a 1:1 stoichiometric ratio, which avoids the formation of other aggregated structures due to insufficient matching of the separated α chains and β chains. The yield is higher and the production is more convenient. No additional chemical cross-linking is required, and the side effects of chemical modification can be avoided. The process is simple and the cost is lower.
(2) The present disclosure uses the nucleic acid encoding the recombinant hemoglobin obtained by tandemly linking four polypeptide chains in the primary structure as a template, and can be directly produced by *in vitro* cell-free synthesis, with fast production speed and low cost.

### BRIEF DESCRIPTION OF THE DRAWINGS

FIG. 1 shows the activity test results of hemoglobin fused with EGFP (scHemoglobin-EGFP) obtained by 6 types of *in vitro* synthesis reactions, expressed as fluorescence values;
FIG. 2 shows the test results of the purification effect of hemoglobin of 6 types of scHemoglobin-EGFP, and the higher the fluorescence value, the higher the amount of protein obtained;
FIG. 3 shows the SDS-PAGE electrophoresis results of protein bands in the elution solutions from the purification of 6 types of scHemoglobin-EGFP;
FIG. 4 shows the SDS-PAGE electrophoresis results of protein bands of hemoglobin (scHemoglobin) without EGFP fusion protein obtained by 6 types of IVTT reactions and purification;
FIGs. 5 and 6 are respectively different results of experiments on the effect of adding hemin to the reaction solution for synthesizing EGFP protein (PC) in an *in vitro* synthesis reaction;
FIG. 7 is a photograph after the following steps: expression and purification of the reaction solution of 15 types of scHemoglobin synthesized in an *in vitro* synthesis reaction with the addition of hemin to a final concentration of 20 µM, followed by buffer exchange into PBS buffer;
FIG. 8 shows the SDS-PAGE electrophoresis results after normal expression purification in an *in vitro* synthesis reaction system without adding a DNA template but adding hemin;
FIG. 9 shows the spectral characteristics of hemoglobin in oxygen-carrying and oxygen-free states disclosed in Reference 1;
FIGs. 10-24 show the spectral characteristics of 15 types of scHemoglobin after binding with hemin in Example 4, respectively, with reference to Reference 1.

### DETAILED DESCRIPTION OF THE PREFERRED EMBODIMENT

Specific embodiments of the present disclosure are described below in conjunction with the accompanying drawings. For the specific methods or materials used in the examples, those skilled in the art can make conventional replacement selections based on the technical ideas of the present disclosure and according to existing technologies, and are not limited to the specific records of the examples of the present disclosure.

The methods used in the examples are all conventional methods unless otherwise specified; the materials, reagents, *etc.* used are commercially available unless otherwise specified.

The definitions of terms used herein are intended to incorporate prior art definitions that are generally accepted in the biotechnology fields for each term. Where appropriate, examples are provided. These definitions apply to the terms used throughout this specification unless otherwise limited in specific cases, either alone or as part of a larger group.

"Recombinant hemoglobin" as used herein refers to a hemoglobin molecule and/or variants thereof having a molecular size of at least about 65 kDa and synthesized by any standard molecular biology technique rather than isolated or purified from any animal or human source.

"Variant" as used herein refers to a polypeptide or polynucleotide sequence that differs from a reference polypeptide or polynucleotide sequence but retains its essential properties. Typically, a variant is very similar to a reference polypeptide or polynucleotide sequence in general and is identical in many regions.

A variant, for example, comprises an amino acid sequence of a parent polypeptide sequence having at least one conservative amino acid substitution; alternatively, a variant may comprise an amino acid sequence of a parent polypeptide sequence having at least one non-conservative amino acid substitution, in which case the non-conservative amino acid substitution preferably does not interfere with or inhibit the biological activity of the functional variant, and the non-conservative amino acid substitution may enhance the biological activity of the variant, such that the biological activity of the variant is increased compared to the parent polypeptide.

When used with reference to a polypeptide or polynucleotide sequence, the term "percent sequence homology" refers to a comparison between a polynucleotide and a polypeptide and is determined by comparing two optimally aligned sequences within a comparison window, wherein the portion of the polynucleotide or polypeptide sequence within the comparison window may contain additions or deletions (*i.e.,* gaps) compared to the reference sequence for optimal alignment of the two sequences (which does not contain additions or deletions). The percentage is calculated by determining the number of positions where the same nucleic acid base or amino acid residue is present in both sequences to produce the number of matching positions, dividing the number of matching positions by the total number of positions in the longer sequence in the comparison window, and multiplying the result by 100 to produce the percent sequence homology. Homology is evaluated using any one of a variety of sequence comparison algorithms and programs known in the art. Such algorithms and programs include, but are by no means limited to, TBLASTN, BLASTP, FASTA, TFASTA and CLUSTALW. In certain embodiments, using the Basic Local Alignment Search Tool ("BLAST") well known in the art, protein and nucleic acid sequence homology is evaluated (see, *e.g.,* Karlin and Altschul, 1990, Proc. Natl. Acad. Sci. USA87:2267-2268; Altschul et al., 1990, J. Mol. Biol. 215:403-410; Altschul et al., 1993, Nature Genetics 3:266-272; Altschul et al., 1997, Nuc. Acids Res. 25:3389-3402).

Heme as used herein refers to ferrous heme, which is an iron porphyrin compound and a prosthetic group of hemoglobin, as well as a prosthetic group of myoglobin, cytochrome, peroxidase, catalase, *etc.*

The "active structure" as used herein refers to the prepared recombinant hemoglobin having the ability to carry oxygen and other functions, and can be prepared as or used as a blood substitute. For example, a protein before hemin is unbound, and as long as it has the ability to carry oxygen and other functions after binding to hemin and can be prepared as a blood substitute, the protein before hemin is unbound is considered to have an active structure.

### 1. Recombinant hemoglobin

The recombinant hemoglobin provided by the present disclosure comprises: four polypeptide chains, and the polypeptide chains are two α chains and two β chains, respectively, and the amino acid sequences of the four polypeptide chains are linked by a linker peptide, wherein the order and position of the linker peptide for linking the amino acid sequences of the four polypeptide chains is not particularly specified, but may be in any order or in any position: that is, the four polypeptide chains are linked to each other in the primary structure by one or more linker peptides.

In a preferred example, three of the linker peptides sequentially link the amino acid sequences of the four polypeptide chains from N-terminus to C-terminus in any order, that is, the four polypeptide chains are linked in tandem, preferably in any one of the following orders: α chain-α chain-β chain-β chain, β chain-β chain-α chain-α chain, β chain-α chain-α chain-β chain, α chain-β chain-α chain-β chain and β chain-α chain-β chain-α chain: "-" is a linker peptide, and the amino acid sequences of the three of the linker peptides can be the same, or only two of them are the same, or all of them are different.

The number of amino acids in the linker peptide is to ensure that the recombinant hemoglobin forms a functional active structure, and secondly, to maintain a certain stability. Preferably, the number of amino acids in the linker peptide is 1-90, 1-30, 1-35 or 1-40.

In one example, the linker peptide comprises two types:
One is used to link two α chains, referred to as a first linker peptide, preferably, the number of amino acids of the first linker peptide is 1-5, and more preferably 1, 2 or 3;
the other is used to link two β chains or one α chain and one β chain, referred to as a second linker peptide, preferably, the number of amino acids of the second linker peptide is any one of 5-90, 5-85, 5-30, 5-35, 5-40, 10-30, 10-35 and 10-40.

In one example, the amino acid of the first linker peptide is 1 glycine.

In one example, the second linker peptide comprises main amino acid residues selected from one or more of G, S, T and A, and the number of the main amino acid residues accounts for at least greater than 50%, 60%, 70%, 80% or 90% of the total number of amino acid residues of the second linker peptide; more preferably, the second linker peptide comprises main amino acid residues selected from one or more of G and S, and the total amount of the main amino acid residues accounts for at least greater than 40%, 45%, 60%, 70% or 80% of the total number of amino acid residues of the second linker peptide. The amino acid sequence of the second linker peptide is, for example, SEQ ID NOs: 10-12.

In one example, the second linker peptide comprises at least a flexible unit.

In one example, the structure of the flexible unit is (GGGGS)a, preferably, a is 1-4. More preferably, the flexible unit is selected from:
(1) GGGGS;
(2) GGGGSGGGGS;
(3) GGGGSGGGGSGGGGSGGGGS.

In one example, the second linker peptide further comprises an amphipathic unit composed of alternating hydrophilic and hydrophobic amino acids; at this time, from N-terminus to C-terminus, the amino acid order of the second linker peptide is: flexible unit-amphipathic unit composed of alternating hydrophilic and hydrophobic amino acids-flexible unit.

The alternating of hydrophilic and hydrophobic amino acids as used herein refers to the following different situations:
(1) a hydrophilic amino acid alternates with a hydrophobic amino acid;
(2) one or more consecutive hydrophilic amino acids form a hydrophilic group, and one or more consecutive hydrophobic amino acids form a hydrophobic group. At this time, the amino acids in each group can be the same or different. For example, in each hydrophilic group, the hydrophilic amino acids can be the same or different; the amino acid composition between different hydrophilic groups or different hydrophobic groups can be the same or different, for example;
in one example, the structure of the amphipathic unit is any one or more of (RADA)b, (KLDL)c, (LK)d and (LD)f, preferably: b is 1-5, c is 1-5, d is 1-10, and f is 1-10.

More preferably, the amphipathic unit is selected from:
(1) RADARADARADARADA;
(1) KLDLKLDLKLDL;
(3) LKLKLKLKLKLK;
(4) LDLDLDLDLDLD.

In one example, the amino acid sequence of the second linker peptide is SEQ ID NOs: 1-6.

In one example, the amino acid sequence of the α chain is SEQ ID NO: 7 or has a percent sequence homology of at least 80%, 85%, 90%, 95% or 99% with SEQ ID NO: 7; the amino acid sequence of the β chain is SEQ ID NO: 8 or has a percent sequence homology of at least 80%, 85%, 90%, 95% or 99% with SEQ ID NO: 8.

2. The present disclosure further provides a nucleic acid, comprising: a nucleotide sequence encoding the above recombinant hemoglobin.

The present disclosure further provides a vector, comprising the above nucleic acid.

The present disclosure further provides a host cell, comprising the above nucleic acid and/or the above vector. Herein, the host cell is derived from a prokaryotic cell or a eukaryotic cell, and further, the cell is selected from the following group: one or more of *Escherichia coli* cells, human cells, Chinese hamster cells, ovarian cells, insect cells, wheat germ cells, rabbit reticulocytes and yeast cells.

Further, the host cell is selected from a yeast cell, and furthermore, the yeast cell is selected from: one or more of *Saccharomyces cerevisiae* and *Kluyveromyces;* in another preferred embodiment, the *Kluyveromyces* is selected from: one or more of *Kluyveromyces lactis, Kluyveromyces marxianus* and *Kluyveromyces dobzhanskii.*

The nucleic acid, vector or host cell provided by the present disclosure can be used in the preparation of recombinant hemoglobin.

The present disclosure further provides an *in vitro* cell-free protein synthesis system, comprising: a cell extract and an mRNA or DNA template encoding the above recombinant hemoglobin.

In one example, the cell extract is an *Escherichia coli* extract or a yeast cell extract, further, the cell extract is derived from the following group: one or more of *Saccharomyces cerevisiae, Pichia pastoris* and *Kluyveromyces;* preferably, the yeast cell extract is derived from *Kluyveromyces,* more preferably one or more of *Kluyveromyces lactis, Kluyveromyces marxianus* and *Kluyveromyces dobzhanskii.*

In one example, the *in vitro* cell-free synthesis system further comprises one or more of the following components: an amino acid mixture, a dNTP, an RNA polymerase, a DNA polymerase, an energy supply system, polyethylene glycol and an aqueous solvent.

3. An *in vitro* cell-free synthesis method for the recombinant hemoglobin, comprising:
providing an *in vitro* cell-free protein synthesis system;
adding an mRNA or DNA template encoding the above recombinant hemoglobin to the *in vitro* cell-free protein synthesis system to perform an *in vitro* synthesis reaction of the single-chain polypeptide.

The volume ratio of the DNA template to the *in vitro* cell-free protein synthesis system is 1:10-1:50, preferably 1:20-1:40, most preferably 1:25-1:35, and particularly preferably 1:30.

In one example, the *in vitro* cell-free protein synthesis system comprises: a cell extract.

In one example, the *in vitro* cell-free protein synthesis system comprises one or more of an amino acid mixture, a dNTP, an RNA polymerase, a DNA polymerase, an energy supply system, polyethylene glycol and an aqueous solvent.

In one example, the cell extract is an *Escherichia coli* extract or a yeast cell extract, further, the cell extract is derived from the following group: one or more of *Saccharomyces cerevisiae, Pichia pastoris* and *Kluyveromyces;* preferably, the yeast cell extract is derived from *Kluyveromyces,* more preferably one or more of *Kluyveromyces lactis, Kluyveromyces marxianus* and *Kluyveromyces dobzhanskii.*

In one example, the yeast cell extract accounts for 50-80% of the total *in vitro* cell-free protein synthesis system.

*In vitro* synthesis reaction refers to the reaction of synthesizing protein in an *in vitro* cell-free synthesis system, including at least the translation process. Including but not limited to IVT reaction (*in vitro* translation reaction), IVTT reaction (*in vitro* transcription and translation reaction), and IVDTT reaction (*in vitro* replication transcription and translation reaction). In the present disclosure, IVTT reaction is preferred. IVTT reaction, corresponding to the IVTT system, is the process of transcribing and translating DNA into protein *in vitro.* Therefore, we also refer to this type of *in vitro* protein synthesis system as D2P system, D-to-P system, D_to_P system, DNA-to-Protein system; the corresponding *in vitro* protein synthesis method is also referred to as D2P method, D-to-P method, D_to_P method, DNA-to-Protein method.

In a preferred embodiment, in the *in vitro* cell-free synthesis method of the present disclosure, the *in vitro* protein synthesis system, template, plasmid, target protein, *in vitro* protein synthesis reaction (incubation reaction), various preparation methods, various detection methods and other technical elements of the present disclosure can also be independently selected from the following documents. Suitable embodiments or implementation methods include but are not limited to CN111484998A, CN106978349A, CN108535489A, CN108690139A, CN108949801 A, CN108642076A, CN109022478A, CN109423496A, CN109423497A, CN109423509A, CN109837293A, CN109971783A, CN109988801A, CN109971775A, CN110093284A, CN110408635A, CN110408636A, CN110551745A, CN110551700A, CN110551785A, CN110819647A, CN110845622A, CN110938649A, CN110964736A, *etc.* Unless otherwise inconsistent with the purpose of the present disclosure, these documents and their cited documents are cited in their entirety and for all purposes.

The present disclosure synthesizes the above recombinant hemoglobin by the above *in vitro* cell-free synthesis method, which has the advantages of short cycle, convenient operation and low cost, and can obtain stable hemoglobin with the above active structure.

Further, the *in vitro* cell-free synthesis method further comprises:
simultaneously adding hemin to the *in vitro* cell-free protein synthesis system or adding hemin after the *in vitro* synthesis reaction to generate recombinant hemoglobin bound to the hemin, preferably, the added hemin comprises trivalent iron.

In the following, the present disclosure is further illustrated by specific examples.

In the following examples:
(1) The IVTT reaction system is: 4-hydroxyethylpiperazineethanesulfonic acid at a final concentration of 22 mM with a pH of 7.4, 30-150 mM potassium acetate, 1.0-5.0 mM magnesium acetate, 1.5-4 mM nucleoside triphosphate mixture (adenosine triphosphate, guanosine triphosphate, cytosine triphosphate and uridine triphosphate), 0.08-0.24 mM amino acid mixture (glycine, alanine, valine, leucine, isoleucine, phenylalanine, proline, tryptophan, serine, tyrosine, cysteine, methionine, asparagine, glutamine, threonine, aspartic acid, glutamic acid, lysine, arginine and histidine), 25 mM creatine phosphate, 1.7 mM dithiothreitol, 0.27 mg/mL creatine phosphate kinase, 0.027-0.054 mg/mL T7 RNA polymerase, 1%-4% polyethylene glycol, 0.5%-2% sucrose, and finally 50-80% volume of yeast cell extract are added.
   The yeast cell extract is derived from *Kluyveromyces.*
(2) The transformation of each target plasmid is carried out as follows:
   1 µL of target plasmid was added to 20 µL of DH5α and placed on ice for 30 min; heat shock was carried out at 42°C for 45 s, and the mixture was placed on ice for 2 min, added with 500 µL of culture medium, and cultured at 37°C with shaking for 1 hour (200 rpm). 100 µL of the mixture was pipetted into an LB (containing antibody) plate, cultured inverted at 37°C for 12-16 hours, taken out and stored in a 4°C refrigerator.

(4) Extraction and amplification of the transformed plasmid:
Amplification system: random primers with a final concentration of 20-30 µM, 0.05-0.15 µg/mL plasmid template, 0.5-1 mM dNTP, 2 × BSA, 1 × phi29 reaction buffer (components are 50 mM Tris-HCl, 10 mM MgCl₂, 10 mM (NH₄)₂SO₄, 4 mM DTT, pH 7.5).

Taking 10 mL of amplification system as an example:
10 mL of amplification system was mixed with plasmid (a final concentration of 4 ng/µL) and 5 µL of phi29 DNA polymerase (0.05-0.1 mg/mL), reacted at 37°C and 30 rpm for 2 hours, and then run on a gel (1% agarose gel) for identification. The obtained product was used as a DNA template.

**In the following examples, unless otherwise specified, the constructed structures are all from N-terminus to C-terminus.**

### Example 1. Expression and purification of hemoglobin fused with EGFP

Different linkers were designed to link the four subunits of hemoglobin, that is, the four polypeptide chains were linked in tandem to form a single-chain scHemoglobin, and the EGFP protein was fused to the C-terminus of scHemoglobin to monitor the expression of the protein. The numbers are hemo006, hemo007, hemo008, hemo009, hemo010, and hemo011, respectively. The corresponding subunit tandem methods are shown in Table 1. In this example, the amino acid sequence of the α chain is SEQ ID NO: 7, and the amino acid sequence of the β chain is SEQ ID NO: 8.

| Table 1 | | | |
|---|---|---|---|
| No. | Subunit tandem order | Linker peptide (linker) (in this example, all linkers between α-α are one glycine) | Molecular weight KDa |
| hemo006 | β-α-α-β | The linker peptides between β-α and α-β are both SEQ ID NO: 1: GGGGSRADARADARADARADAGGGGS | 97.5 |
| hemo007 | β-α-α-β | The linker peptides between β-α and α-β are both SEQ ID NO: 2: GGGGSKLDLKLDLKLDLGGGGS | 97.1 |
| hemo008 | β-α-α-β | The linker peptide between β-α is SEQ ID NO: 3: GGGGSLKLKLKLKLKLKGGGGS; | 97.1 |
| | | the linker peptide between α-β is SEQ ID NO: 4: GGGGSLDLDLDLDLDLDGGGGS | |
| hemo009 | β-α-α-β | The linker peptides between β-α and α-β are both SEQ ID NO: 5: GGGGSGGGGS | 94.2 |
| hemo010 | β-β-α-α | The linker peptides between β-β and β-α are both SEQ ID NO: 5: GGGGSGGGGS | 94.2 |
| hemo011 | β-α-α-β | The linker peptides between β-α and α-β are both SEQ ID NO: 6: GGGGSGGGGSGGGGSGGGGS | 96.4 |

Through gene synthesis, single-chain hemoglobin genes containing different linker peptides were synthesized and directly inserted into the optimized plasmid (D2P1.08t) of our company, with the insertion sites being BamHI and HindIII. A total of 6 plasmids containing target gene fragments, hemo006-hemo011, were obtained, and DNA templates encoding the structure in Table 1 were obtained by transformation and amplification.

15 ng/µL DNA templates were added to the IVTT reaction system respectively. After mixing well, the mixture was placed in an environment of 25-30°C and reacted for 3 hours. After the reaction was completed, the reaction solution was collected, and the RFU fluorescence value of the EGFP fusion protein was read using a microplate reader.

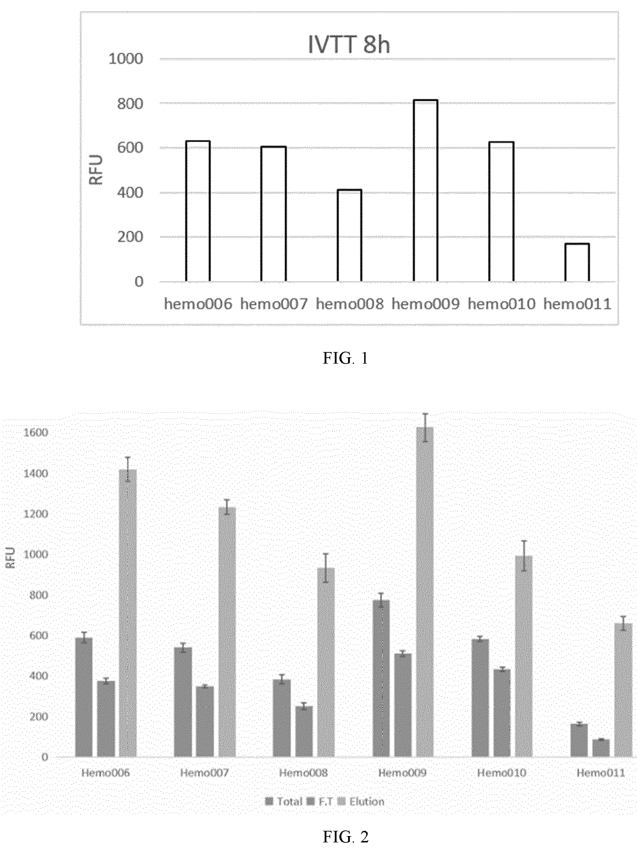

The products of the above 6 types of proteins synthesized and expressed by IVTT were subjected to fluorescence detection, confirming that the hemoglobins of these 6 fusion methods could be expressed normally. As shown in FIG. 1.

After verifying that these scHemoglobin-EGFP fusion proteins could be expressed normally, the reaction solution after the IVTT reaction was purified to test the purification effect. 1 mL of the reaction solution after the IVTT reaction was added with nickel magnetic beads for purification, and the fluorescence values of the flow-through solution and elution solution were recorded. The purification effect is shown in FIG. 2. The results show that these 6 types of scHemoglobin-EGFP design schemes can be purified to obtain the target protein. In FIG. 2, the fluorescence values of each protein from left to right are: the reaction solution after the IVTT reaction (total, that is, before adding magnetic beads), the flow-through solution (FT) after binding with magnetic beads, and the final elution solution (Elution).

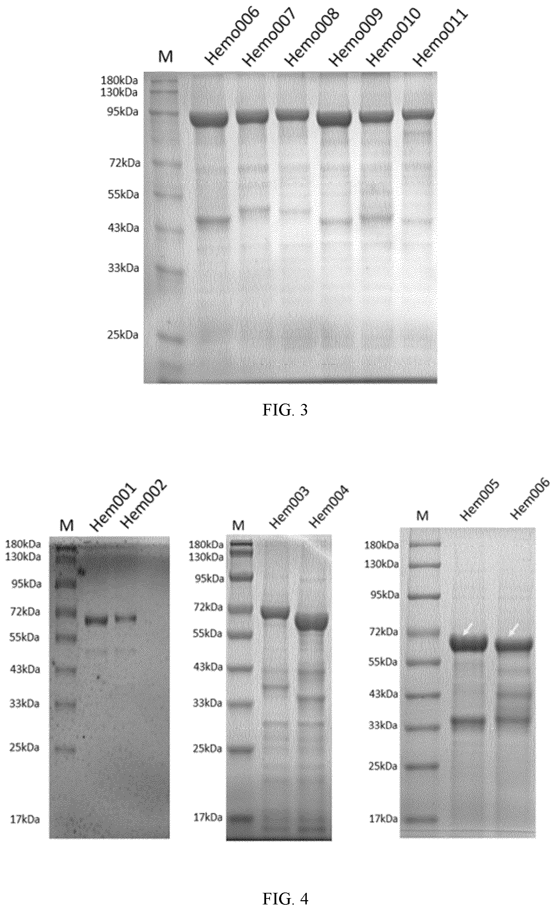

The elution solution was subjected to SDS-PAGE electrophoresis to detect protein bands. The results are shown in FIG. 3, which shows that the protein size is consistent with the theoretical molecular weight.

### Example 2. Expression and purification of scHemoglobin

scHemoglobin was constructed alone without EGFP fusion protein. Based on Example 1, 6 types of scHemoglobin were constructed, and the construction methods are shown in Table 2:

| Table 2 | | | |
|---|---|---|---|
| No. | Subunit tandem order | Linker peptide (linker) (in this example, all linkers between α-α are one glycine) | Molecular weight KDa |
| HEM001 | β-α-α-β | The linker peptides between β-α and α-β are both SEQ ID NO: 1: | 70.3 |
| | | GGGGSRADARADARADARADAGGGGS | |
| HEM002 | β-α-α-β | The linker peptides between β-α and α-β are both SEQ ID NO: 2: GGGGSKLDLKLDLKLDLGGGGS | 69.8 |
| HEM003 | β-α-α-β | The linker peptide between β-α is SEQ ID NO: 3: GGGGSLKLKLKLKLKLKGGGGS; | 69.8 |
| | | the linker peptide between α-β is SEQ ID NO: 4: GGGGSLDLDLDLDLDLDGGGGS | |
| HEM004 | β-α-α-β | The linker peptides between β-α and α-β are both SEQ ID NO: 5: GGGGSGGGGS | 67 |
| HEM005 | β-β-α-α | The linker peptides between β-β and β-α are both SEQ ID NO: 5: GGGGSGGGGS | 67 |
| HEM006 | β-α-α-β | The linker peptides between β-α and α-β are both SEQ ID NO: 6: GGGGSGGGGSGGGGSGGGGS | 68.3 |

According to the same method as Example 1, the rest was the same as Example 1, except that EGFP was not fused, and after obtaining the DNA template, without adding heme, the IVTT reaction system was used for reaction and purification, and the obtained protein was verified by SDS-PAGE electrophoresis as shown in FIG. 4, and the results show that scHemoglobin can be correctly expressed.

### Example 3. Co-expression and purification of scHemoglobin and hemin

Since natural hemoglobin needs to be combined with heme to exert its oxygen-carrying capacity, the artificial hemoglobin we produce also needs to be combined with heme to function normally. However, since heme binds to divalent iron ions, and divalent iron ions are very unstable and easily oxidized to trivalent iron ions in the air. Heme divalent iron ions can only exist stably when bound to hemoglobin. Therefore, it is currently difficult to buy a large amount of heme that is not bound to hemoglobin on the market. Heme sold on the market is generally not in stock, has a long delivery time, and is expensive.

We tried to use hemin as the prosthetic group of scHemoglobin. Hemin is hemin comprises trivalent iron.

First, the possible impact of the concentration of hemin added to the IVTT reaction system was tested:

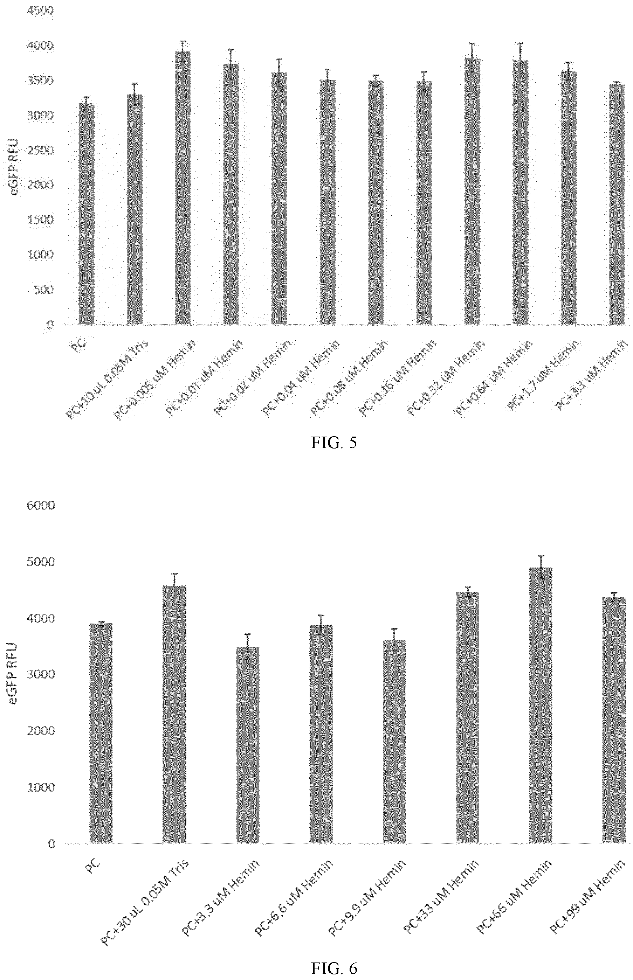

Different concentrations of hemin were added to the IVTT reaction solution for EGFP protein expression synthesis, and the expression level of EGFP protein was tested to detect whether the addition of hemin affected the expression level of PC. The results showed that hemin with a final concentration of 3.3 µM did not affect the IVTT expression level of PC, as shown in FIG. 5; the concentration of hemin was then increased and the test was continued, as shown in FIG. 6. Hemin with a final concentration of 99 µM did not affect the IVTT expression level of PC.

That is, the amount of hemin added had no effect on the expression of the IVTT reaction, and there was no particular restriction on the amount added, which was mainly higher than the expected yield of hemoglobin to ensure that all expressed hemoglobin was in a hemin-bound state.

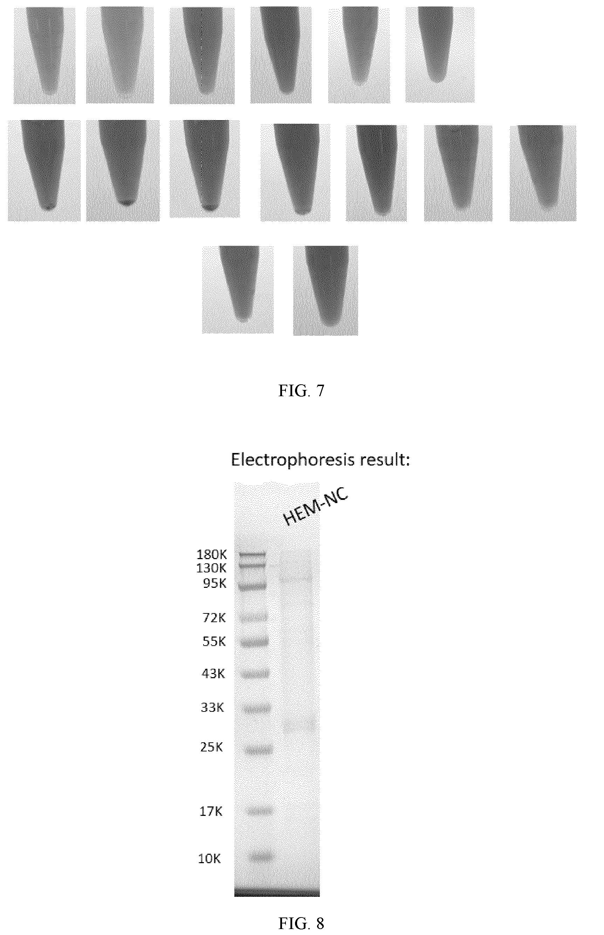

Then, the construction structures shown in Tables 2 and 3 were used for scHemoglobin expression and synthesis, and hemin with a final concentration of 20 µM was added to the synthesized IVTT reaction solution for expression and purification. The results corresponding to each construction were run on a gel, and the results were consistent with the molecular weight, indicating that the target protein was obtained. Moreover, after replacing each purified protein corresponding to the various constructions into PBS solution, the proteins displayed a blood red color (as shown in FIG. 7, the first row, from left to right, are the pictures corresponding to the structures constructed by HM001, HM002, HM003, HM004, HM005 and HEM006 after binding with hemin; the second row, from left to right, are HM007, HM008, HM009, HEM010, HM013, HM014 and HEM015; the third row, from left to right, are HEM023 and HEM024).

| Table 3 | | | |
|---|---|---|---|
| No. | Subunit tandem order | Linker peptide (linker) (in this example, all linkers between α-α are one glycine) | Note |
| HEM007 | β-α-α-β | The linker peptides between β-α and α-β are both SEQ ID NO: 10: SGGGSGGGSEGGGSEGGGSE GGGSEGGGSGGGSG | The amino acid sequence of the α chain is SEQ ID NO: 7; the amino acid sequence of the β chain is SEQ ID NO: 8 |
| HEM008 | β-α-α-β | The linker peptides between β-α and α-β are both SEQ ID NO: 11: | |
| | | GGSSGSGSGSTGTSSSGTGTS AGTTGTSASTSGSGSGGGGGS GGGGSAGGTATAGASSGS | |
| HEM009 | β-α-α-β | The linker peptides between β-α and α-β are both SEQ ID NO: 12: | |
| | | GGSSGSGSGSTGTSSSGTGTS AGTTGTSASTSGSGSGGGGGS GGGGSAGGTATAGAGSTGGA GSTGGAGSTGGAGTGSGAG | |
| HEM010 | β-α-α-β (ba bovine ab) | The linker peptides between β-α and α-β are both SEQ ID NO: 1: | From left to right, in order: The amino acid sequence of the left terminal β chain is SEQ ID NO: 8; |
| | | GGGGSRADARADARADARA DAGGGGS | the amino acid sequence of the first α chain is SEQ ID NO: 13; |
| | | | the amino acid sequence of the first α chain is SEQ ID NO: 7; the amino acid sequence of the right terminal β chain is: SEQ ID NO: 8 |
| HEM013 | β-α-α-β (b pig aab) | GGGGSRADARADARADARA DAGGGGS The linker peptides between β-α and α-β are both SEQ ID NO: 1: | From left to right, in order: The amino acid sequence of the left terminal β chain is: SEQ ID NO: 16; |
| | | | the amino acid sequence of the two α chains is SEQ ID NO: 7; the amino acid sequence of the right terminal β chain is: SEQ ID NO: 8 |
| HEM014 | β-α-α-β (ba pig ab) | The linker peptides between β-α and α-β are both SEQ ID NO: 1: | From left to right, in order: The amino acid sequences of |
| | | GGGGSRADARADARADARA DAGGGGS | the two β chains are: SEQ ID NO: 8; |
| | | | the amino acid sequence of the first α chain is SEQ ID NO: 14; |
| | | | the amino acid sequence of the second α chain is SEQ ID NO: 7 |
| HEM015 | β-α-α-β (b pig a pig ab) | The linker peptides between β-α and α-β are both SEQ ID NO: 1: | From left to right, in order: The amino acid sequence of the left terminal β chain is SEQ ID NO: 16; |
| | | GGGGSRADARADARADARA DAGGGGS | the amino acid sequence of the first α chain is SEQ ID NO: 14; |
| | | | the amino acid sequence of the second α chain is SEQ ID NO: 7; |
| | | | the amino acid sequence of the right terminal β chain is: SEQ ID NO: 8 |
| HEM023 | β-α-α-β (bovine source) | The linker peptides between β-α and α-β are both SEQ ID NO: 1: | The amino acid sequences of both α chains are SEQ ID NO: 13; |
| | | GGGGSRADARADARADARA DAGGGGS | |
| | | | the amino acid sequences of the two β chains are SEQ ID NO: 15 |
| HEM024 | β-α-α-β (pig source) | The linker peptides between β-α and α-β are both SEQ ID NO: 1: | The amino acid sequences of both α chains are SEQ ID NO: 14; |
| | | GGGGSRADARADARADARA DAGGGGS | |
| | | | the amino acid sequences of the two β chains are SEQ ID NO: 16 |

Because hemoglobin itself is colorless, blood red is the color of hemin, in order to exclude: after the final purification, free hemin will be obtained in the final product. We conducted the following control experiment (HEM-NC): During the IVTT reaction, no DNA template was added, but hemin was added to perform normal expression purification. The obtained protein elution solution was also subjected to buffer replacement to exchange the original buffer with PBS buffer. SDS-PAGE electrophoresis and color observation showed no scHemoglobin electrophoresis band (results shown in FIG. 8), and after replacement with PBS buffer, the solution was colorless and transparent, without showing blood red. This indicates that the blood red sample we obtained before is the color that can only be presented after the protein binds to hemin.

### Example 4. Oxygen-carrying experiment

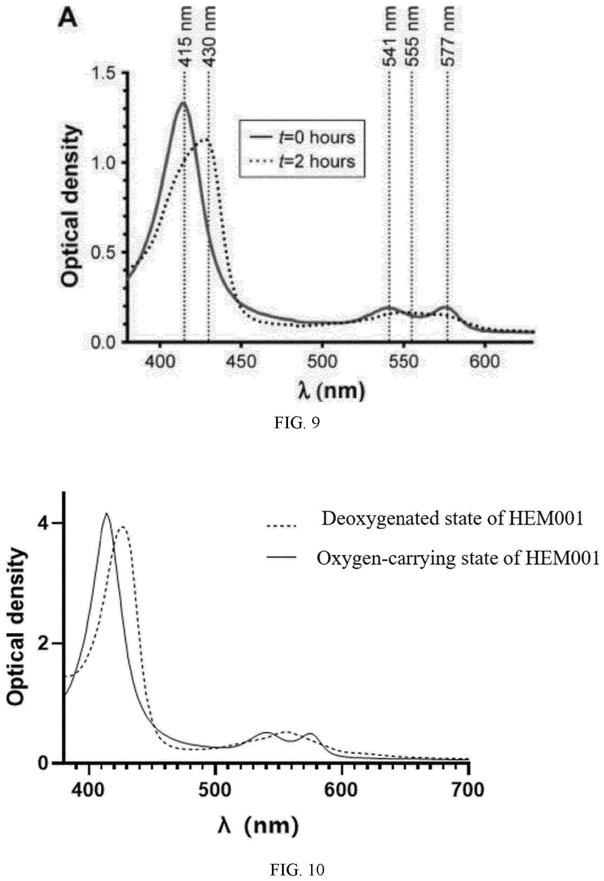

The proteins corresponding to the constructions in Tables 2 and 3 obtained by purification according to Example 3 were subjected to oxygen-carrying experiments, as follows:
The spectral characteristics of hemoglobin in oxygen-carrying and oxygen-free states follow FIG. 9 (Reference 1: Patel, Mira P et al. "Development and validation of an oxygen dissociation assay, a screening platform for discovering, and characterizing hemoglobin-oxygen affinity modifiers." Drug design, development and therapy 2018, vol. 12 1599-1607):
As shown in FIG. 9, in the oxygen-carrying state, hemoglobin peaks at 415, 541 and 577 nm. In FIG. 9, it can be clearly seen that the absorbance of hemoglobin at 415 nm in the oxygen-carrying state is higher than the absorbance of hemoglobin at 430 nm in the oxygen-free state. In the oxygen-free state, hemoglobin peaks at 430 nm and 555 nm. In FIG. 9, it can be clearly seen that the absorbance of hemoglobin at 430 nm in the oxygen-free state is higher than the absorbance of hemoglobin at 430 nm in the oxygen-carrying state.

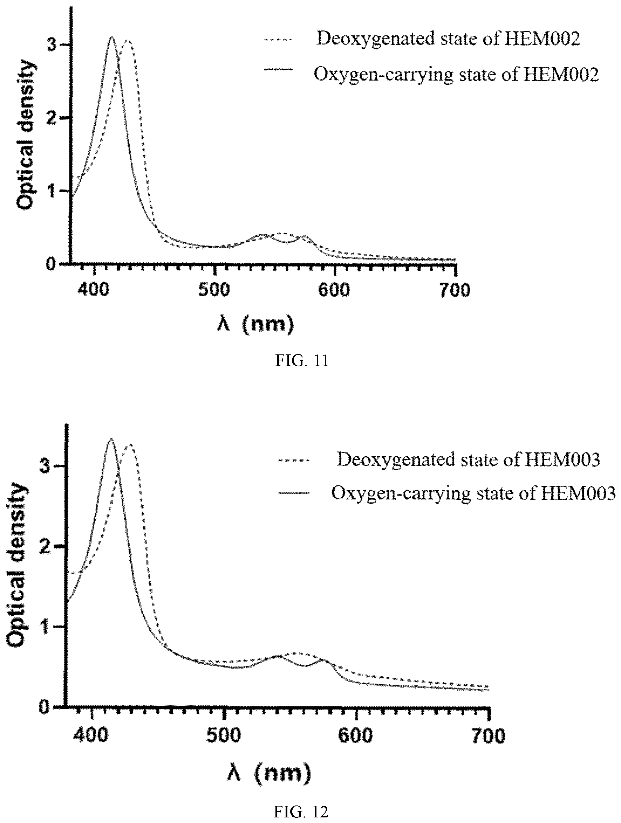

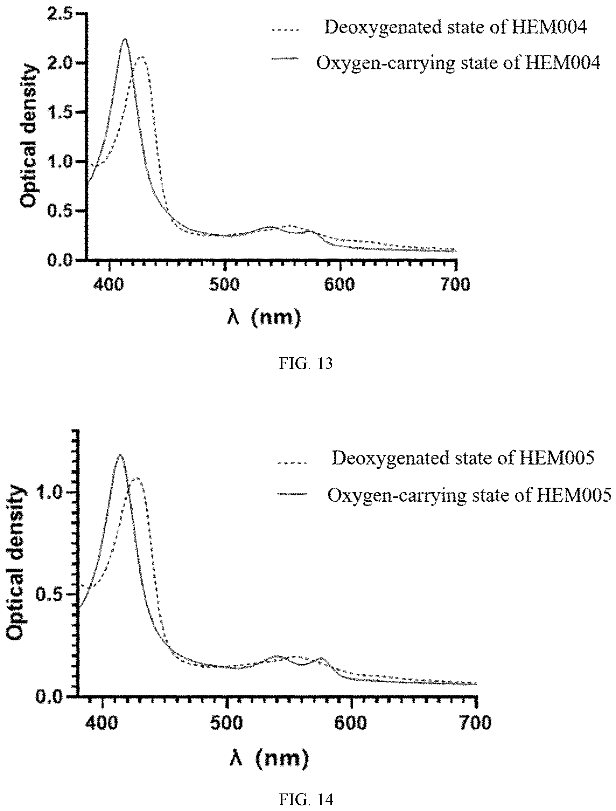

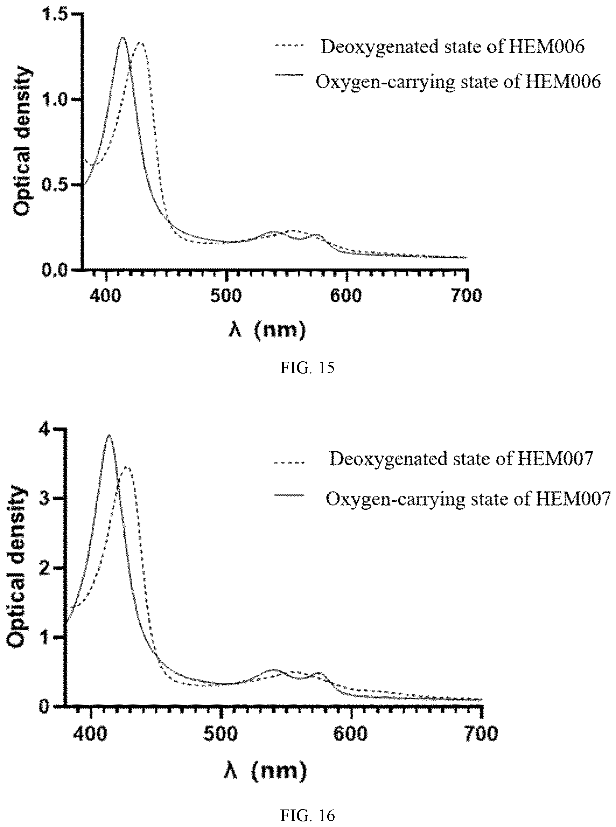

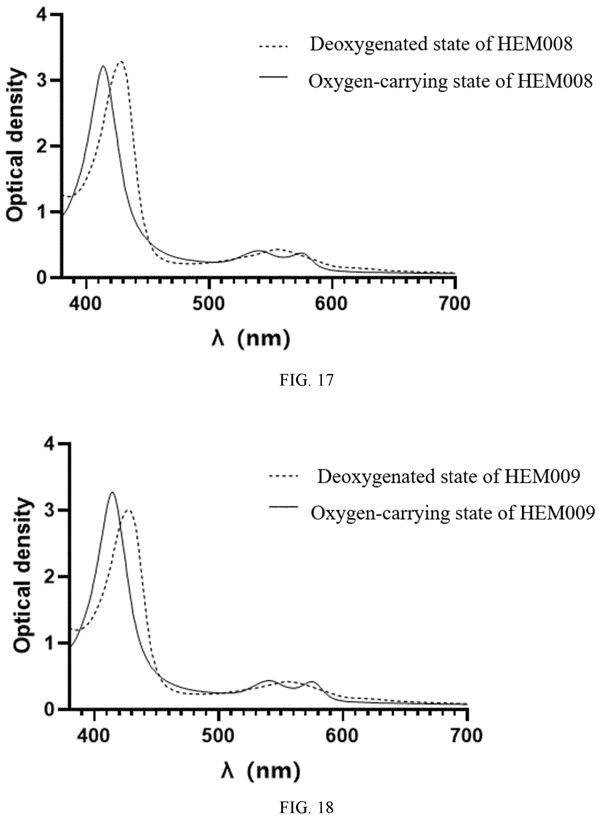

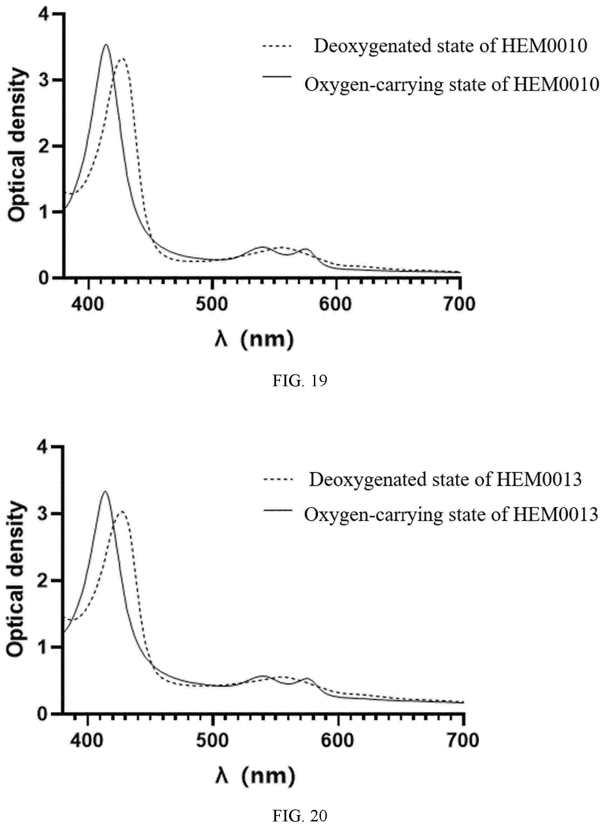

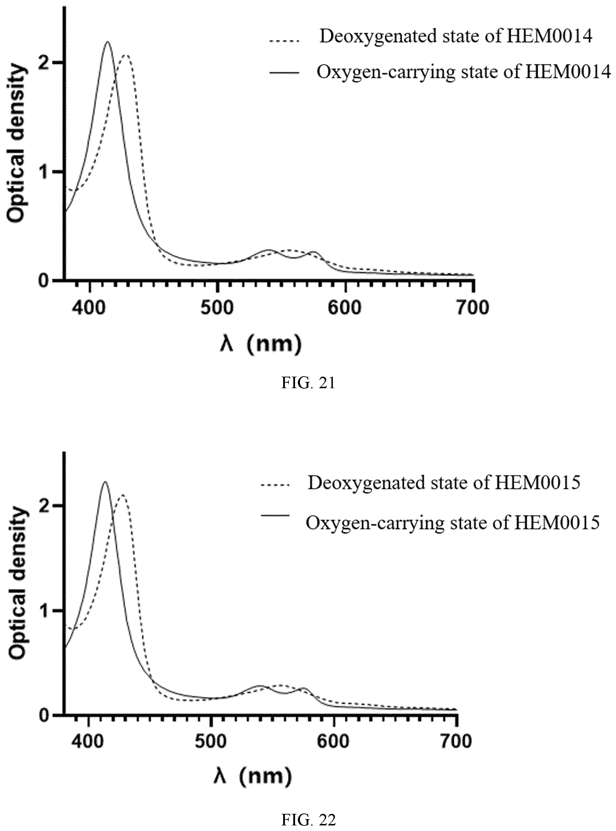

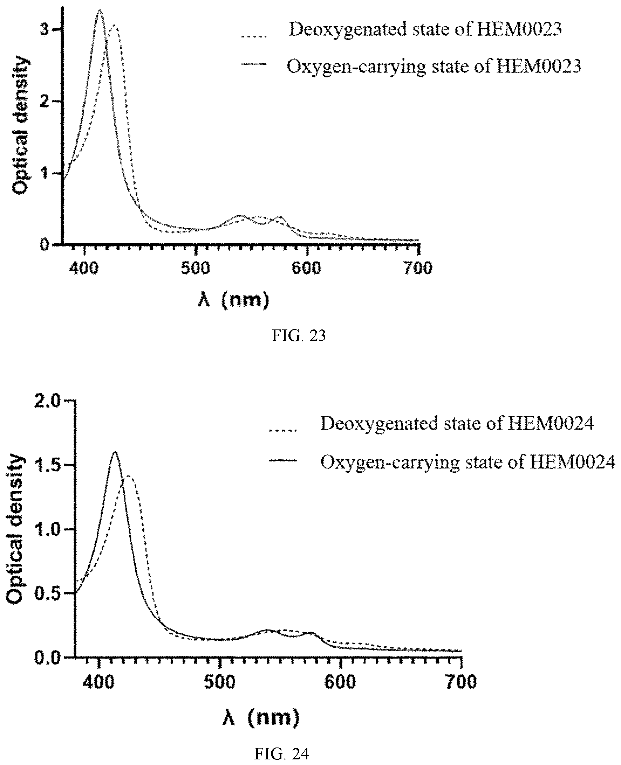

The conversion of hemoglobin from the oxygen-carrying state to the oxygen-free state is catalyzed by sodium dithionite: after sodium dithionite is added to hemoglobin in the oxygen-carrying state, the hemoglobin is reduced, losing oxygen at the same time, turning into a deoxygenated state, and the spectral characteristics are transformed. Based on this, for the proteins obtained after binding hemin corresponding to each of the constructions of Tables 2 and 3 obtained in accordance with Example 3, the results of spectral detection are shown in FIGs. 10-24:

As can be seen from FIGs. 10-24, the spectral characteristics of the spectral detection of the 15 hemin-bound proteins obtained in Example 3 are consistent with the schematic diagram of FIG. 9, that is, it is confirmed that the present disclosure can obtain functional hemoglobin by linking the amino acid sequences of the four polypeptide chains through linker peptides.

### Example 5. Stability test

The stability of the protein is determined by the Tm value. Tm is measured by the Uncle protein stability analyzer of Unchained.

The intrinsic fluorescence of the protein comes from amino acids containing benzene rings (tryptophan, tyrosine and phenylalanine), which emit photons that can be absorbed *(i.e.,* fluorescence) under ultraviolet wavelength excitation. As the environment of these aromatic amino acids in the protein is different, the emitted fluorescence intensity is also different. Therefore, the changing trend of the intrinsic fluorescence can be used to indicate the conformational changes of the tertiary structure of the protein, thereby analyzing the conformational stability of the protein.

By detecting the change of the fluorescence signal during the heating process, a thermal denaturation curve can be obtained, which describes one or more phase transitions when the protein denatures. The midpoint of this transition (Tm) can be used to describe the stability of the protein. The higher the Tm, the higher the protein stability.

The Tm value of the same hemoglobin used in the oxygen-carrying experiment as in Example 4 was tested, and the average Tm value was not much different from the Tm value of the bovine hemoglobin in the control group, indicating that the stability of the modified hemoglobin maintained the level of the original protein.

The sequences mentioned above herein are summarized in Table 4.

| Table 4 | | |
|---|---|---|
| Name | Sequence number | Amino acid sequence |
| Second linker peptide | SEQ ID NO: 1 | GGGGSRADARADARADARADAGGGGS |
| Second linker peptide | SEQ ID NO: 2 | GGGGSKLDLKLDLKLDLGGGGS |
| Second linker peptide | SEQ ID NO: 3 | GGGGSLKLKLKLKLKLKGGGGS |
| Second linker peptide | SEQ ID NO: 4 | GGGGSLDLDLDLDLDLDGGGGS |
| Second linker peptide | SEQ ID NO: 5 | GGGGSGGGGS |
| Second linker peptide | SEQ ID NO: 6 | GGGGSGGGGSGGGGSGGGGS |
| Hemoglobin α polypeptide chain | SEQ ID NO: 7 | VLSPADKTNVKAAWAKVGAHAGEYGAEAFERMFLSF PTTKTYFPHFDLSHGSAQVKGQGKKVADALTNAVAH VDDMPNALSALSDLHAHKLRVDPWNFKLLSHCLLVT LAAHLPAEFTPAVHASLDKFLASVSTVLTSKYR |
| Hemoglobin β polypeptide chain | SEQ ID NO: 8 | VHLTPEEKSAVTALWAKVNVDEVGGEALGRLLVVYP WTQRFFEHFGDLSTPDAVMGNPKVKAHGKKVLGAFS DGLAHLDNLDGTFATLSELHCDKLHVDPENFRLLGNV LVCVLAIHFGKEFTPPVQAAYQKVVAGVANALAHKY H |
| EGFP | SEQ ID NO: 9 | VSKGEELFTGVVPILVELDGDVNGHKFSVRGEGEGDA TNGKLTLKFICTTGKLPVPWPTLVTTLTYGVQCFSRYP DHMKQHDFFKSAMPEGYVQERTISFKDDGTYKTRAE VKFEGDTLVNRIELKGIDFKEDGNILGHKLEYNFNSHN VYITADKQKNGIKANFKIRHNVEDGSVQLADHYQQN TPIGDGPVLLPDNHYLSTQSKLSKDPNEKRDHMVLLE FVTAAGITLGMDELYK |
| Second linker peptide | SEQ ID NO: 10 | SGGGSGGGSEGGGSEGGGSEGGGSEGGGSGGGSG |
| Second linker peptide | SEQ ID NO: 11 | GGSSGSGSGSTGTSSSGTGTSAGTTGTSASTSGSGSGG GGGSGGGGSAGGTATAGASSGS |
| Second linker peptide | SEQ ID NO: 12 | GGSSGSGSGSTGTSSSGTGTSAGTTGTSASTSGSGSGG GGGSGGGGSAGGTATAGAGSTGGAGSTGGAGSTGGA GTGSGAG |
| Hemoglobin α polypeptide | SEQ ID NO: 13 | VLSAADKGNVKAAWGKVGGHAAEYGAEALERMFLS FPTTKTYFPHFDLSHGSAQVKGHGAKVAAALTKAVEH |
| chain | | LDDLPGALSELSDLHAHKLRVDPVNFKLLSHSLLVTL ASHLPSDFTPAVHASLDKFLANVSTVLTSKYR |
| Hemoglobin α polypeptide chain | SEQ ID NO: 14 | VLSAADKANVKAAWGKVGGQAGAHGAEALERMFL GFPTTKTYFPHFNLSHGSDQVKAHGQKVADALTKAV GHLDDLPGALSALSDLHAHKLRVDPVNFKLLSHCLLV TLAAHHPDDFNPSVHASLDKFLANVSTVLTSKYR |
| Hemoglobin β polypeptide chain | SEQ ID NO: 15 | LTAEEKAAVTAFWGKVKVDEVGGEALGRLLVVYPWT QRFFESFGDLSTADAVMNNPKVKAHGKKVLDSFSNG MKHLDDLKGTFAALSELHCDKLHVDPENFKLLGNVL VVVLARNFGKEFTPVLQADFQKVVAGVANALAHRYH |
| Hemoglobin β polypeptide chain | SEQ ID NO: 16 | VHLSAEEKEAVLGLWGKVNVDEVGGEALGRLLVVYP WTQRFFESFGDLSNADAVMGNPKVKAHGKKVLQSFS DGLKHLDNLKGTFAKLSELHCDQLHVDPENFRLLGN VIVVVLARRLGHDFNPNVQAAFQKVVAGVANALAHK YH |

## Claims

1. A recombinant hemoglobin, comprising:
four polypeptide chains, and the polypeptide chains are two α chains and two β chains, respectively,
wherein the amino acid sequences of the four polypeptide chains are linked by linker peptides.

2. The recombinant hemoglobin according to claim 1, wherein
three of the linker peptides sequentially link the amino acid sequences of the four polypeptide chains from N-terminus to C-terminus in any order.

3. The recombinant hemoglobin according to claim 1 or 2, wherein
the number of amino acids in the linker peptide is 1-90, 1-30, 1-35 or 1-40.

4. The recombinant hemoglobin according to claim 3, wherein
the linker peptide used to link the two α chains is a first linker peptide, and the number of amino acids in the first linker peptide is 1-5, preferably 1, 2 or 3,
the linker peptide used to link the two β chains or one α chain and one β chain is a second linker peptide, and the number of amino acids in the second linker peptide is 5-90, 5-85, 5-30, 5-35, 5-40, 10-30, 10-35 or 10-40.

5. The recombinant hemoglobin according to claim 4, wherein
the amino acid of the first linker peptide is 1 glycine.

6. The recombinant hemoglobin according to claim 4 or 5, wherein
the second linker peptide comprises main amino acid residues selected from one or more of G, S, T and A, and the number of the main amino acid residues accounts for at least greater than 50%, 60%, 70%, 80% or 90% of the total number of amino acid residues of the second linker peptide; more preferably, the second linker peptide comprises main amino acid residues selected from one or more of G and S, and the total amount of the main amino acid residues accounts for at least greater than 40%, 45%, 60%, 70% or 80% of the total number of amino acid residues of the second linker peptide.

7. The recombinant hemoglobin according to claim 4 or 5, wherein
the second linker peptide comprises at least a flexible unit.

8. The recombinant hemoglobin according to claim 7, wherein
the second linker peptide further comprises an amphipathic unit composed of alternating hydrophilic and hydrophobic amino acids; at this time, from N-terminus to C-terminus, the amino acid order of the second linker peptide is: flexible unit-amphipathic unit composed of alternating hydrophilic and hydrophobic amino acids-flexible unit.

9. The recombinant hemoglobin according to claim 7 or 8, wherein
the structure of the flexible unit is (GGGGS)a,
preferably, a is 1-4,
more preferably, the flexible unit is selected from:
(1) GGGGS;
(2) GGGGSGGGGS;
(3) GGGGSGGGGSGGGGSGGGGS.

10. The recombinant hemoglobin according to claim 8 or 9, wherein
the structure of the amphipathic unit is any one or more of (RADA)b, (KLDL)c, (LK)d and (LD)f,
preferably: b is 1-5, c is 1-5, d is 1-10, and f is 1-10,
more preferably, the amphipathic unit is selected from:
(1) RADARADARADARADA;
(2) KLDLKLDLKLDL;
(3) LKLKLKLKLKLK;
(4) LDLDLDLDLDLD.

11. The recombinant hemoglobin according to any one of claims 4, 5 and 7-1, wherein
the amino acid sequence of the second linker peptide is SEQ ID NOs: 1-6.

12. The recombinant hemoglobin according to any one of claims 1-11, wherein
the amino acid sequences of the four polypeptide chains are linked from N-terminus to C-terminus in any one of the following orders:
α chain-α chain-β chain-β chain, β chain-β chain-α chain-α chain, β chain-α chain-α chain-β chain, α chain-β chain-α chain-β chain and β chain-α chain-β chain-α chain.

13. The recombinant hemoglobin according to any one of claims 1-12, wherein
the α chain is derived from any one or more of human, pig or bovine species, and the β chain is derived from any one or more of human, pig or bovine species; and/or
the amino acid sequence of the α chain is any one or more of SEQ ID NOs: 7, 13 and 14, or has a percent sequence homology of at least 80%, 85%, 90%, 95%, 97% or 99% compared with any one or more of SEQ ID NOs: 7, 13 and 14, respectively;
the amino acid sequence of the β chain is any one or more of SEQ ID NOs: 8, 15 and 16, or has a percent sequence homology of at least 80%, 85%, 90%, 95%, 97% or 99% compared with any one or more of SEQ ID NOs: 8, 15 and 16, respectively.

14. The recombinant hemoglobin according to any one of claims 1-11, further comprising:
hemin bound to each polypeptide chain respectively.

15. A nucleic acid, comprising:
a nucleotide encoding the recombinant hemoglobin as defined in any one of claims 1-14.

16. A vector, comprising: the nucleic acid as defined in claim 15.

17. A host cell, comprising: the nucleic acid as defined in claim 15 and/or the vector as defined in claim 16,
preferably, the host cell is derived from a prokaryotic cell or a eukaryotic cell,
further, the host cell is derived from bacteria, mammalian cells, human cells, plant cells, yeast cells or insect cells, preferably selected from *Escherichia coli* or yeast cells, and furthermore, the yeast cell is selected from: one or more of *Saccharomyces cerevisiae* and *Kluyveromyces;* in another preferred embodiment, the *Kluyveromyces* is selected from: one or more of *Kluyveromyces lactis, Kluyveromyces marxianus* and *Kluyveromyces dobzhanskii.*

18. Use of the nucleic acid as defined in claim 15, the vector as defined in claim 16 or the host cell as defined in claim 17 in the preparation of the recombinant hemoglobin as defined in any one of claims 1-14.

19. An *in vitro* cell-free protein synthesis system, comprising:
a cell extract; and
an mRNA or DNA template encoding the recombinant hemoglobin as defined in any one of claims 1-14, preferably, the cell extract is an *Escherichia coli* extract or a yeast cell extract, further, the cell extract is derived from the following group: one or more of *Saccharomyces cerevisiae, Pichia pastoris* and *Kluyveromyces;* preferably, the yeast cell extract is derived from *Kluyveromyces,* more preferably one or more of *Kluyveromyces lactis, Kluyveromyces marxianus* and *Kluyveromyces dobzhanskii;*
furthermore, the *in vitro* cell-free protein synthesis system further comprises one or more of the following components:
an amino acid mixture, a dNTP, an RNA polymerase, a DNA polymerase, an energy supply system, polyethylene glycol and an aqueous solvent.

20. An *in vitro* cell-free synthesis method for the recombinant hemoglobin as defined in any one of claims 1-14, comprising:
providing an *in vitro* cell-free protein synthesis system;
adding an mRNA or DNA template encoding the recombinant hemoglobin as defined in any one of claims 1-14 to the *in vitro* cell-free protein synthesis system to perform an *in vitro* synthesis reaction to obtain the recombinant hemoglobin, preferably, the volume ratio of the DNA template to the *in vitro* cell-free protein synthesis system is 1:10 to 1:50, preferably 1:20 to 1:40, most preferably 1:25 to 1:35, particularly preferably 1:30.

21. The *in vitro* cell-free synthesis method according to claim 20, further comprising:
adding hemin to the *in vitro* cell-free protein synthesis system to generate recombinant hemoglobin bound to the hemin, preferably, the added hemin comprises trivalent iron.

22. The *in vitro* cell-free synthesis method according to claim 20 or 21, wherein
the *in vitro* cell-free protein synthesis system comprises: a cell extract;
further,
the *in vitro* cell-free protein synthesis system comprises one or more of an amino acid mixture, a dNTP, an RNA polymerase, a DNA polymerase, an energy supply system, polyethylene glycol and an aqueous solvent; and/or
the cell extract is an *Escherichia coli* extract or a yeast cell extract, further, the cell extract is derived from the following group: one or more of *Saccharomyces cerevisiae, Pichia pastoris* and *Kluyveromyces;* preferably, the yeast cell extract is derived from *Kluyveromyces,* more preferably one or more of *Kluyveromyces lactis, Kluyveromyces marxianus* and *Kluyveromyces dobzhanskii;* or
the yeast cell extract accounts for 50-80% of the entire *in vitro* cell-free protein synthesis system.

23. Use of the recombinant hemoglobin as defined in any one of claims 1-14 as a blood substitute, and for cancer, stroke, hemorrhagic shock, acute mountain sickness (AMS), peripheral arterial disease (PAD) and Parkinson's disease (PD).
